(19) Europäisches Patentamt / European Patent Office / Office européen des brevets

(11) **EP 2 030 571 A1**

(12) **EUROPEAN PATENT APPLICATION**
published in accordance with Art. 153(4) EPC

(43) Date of publication:
**04.03.2009 Bulletin 2009/10**

(51) Int Cl.:
***A61B 8/08*** *(2006.01)*

(21) Application number: **06757261.0**

(22) Date of filing: **12.06.2006**

(86) International application number:
**PCT/JP2006/311776**

(87) International publication number:
**WO 2007/144933 (21.12.2007 Gazette 2007/51)**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(71) Applicants:
• **Shimadzu Corporation**
**Kyoto-shi, Kyoto 604-8511 (JP)**
• **Media Cross Co., Ltd**
**Shinjuku-ku**
**Tokyo, 160-0012 (JP)**

(72) Inventors:
• **KATOH, Jun-ichi**
**Kyoto-shi Kyoto 604-8511 (JP)**
• **ITOH, Masao**
**Tokyo 160-0012 (JP)**

(74) Representative: **Wilhelms · Kilian & Partner**
**Patentanwälte**
**Eduard-Schmid-Strasse 2**
**81541 München (DE)**

(54) **ULTRASONIC DIAGNOSIS DEVICE**

(57) An ultrasonograph having a vascular diameter measurement means for taking an ultrasonic tomography image of a blood vessel and then measuring a vascular diameter based on the image, including: an ultrasonic probe 11 having an ultrasonic oscillator 11a for an ultrasonic beam scan, the oscillator being rotatable in the direction orthogonal to the ultrasonic beam's scan direction; a multi-section imaging means for tilting the oscillator 11a at predetermined intervals to perform an ultrasonic beam scan by the oscillator 11a at each position in order to take a tomography image of the blood vessel 50 at a plurality of sections; an imaging position determiner for comparing the results of the measurement of the vascular diameter based on each taken tomography image, and determining the position where an image presenting the largest vascular diameter has been taken as the largest vascular diameter imaging position; and a largest vascular diameter acquisition means for tilting the oscillator 11a to the position determined by the imaging position determiner. This saves the trouble of manually fixing the probe's displacement due to a rapid decompression in releasing the avascularization, which achieves a smooth %FMD measurement.

Fig. 3

**Description**

TECHNICAL FIELD

The present invention pertains to an ultrasonograph, and particularly to an ultrasonograph preferably used for the measurement of a vascular diameter.

BACKGROUND ART

Since arteriosclerosis causes examples of heart disease such as angina pectoris and myocardial infarction, cerebral infarction, and other diseases, it is preferable to regularly perform an examination. One known method for noninvasively assessing the pliancy of a blood vessel for an early diagnosis of arteriosclerosis is a flow mediated dilation (FMD) test using an ultrasonograph (refer to Patent Document 1 for example).

A flow mediated dilation is a reaction in which an increase in blood flow generates a shear stress which acts on the vascular endothelium, causing nitric monoxide which is a vasodilating agent to generate from an endocapillary cell and dilate the vascular diameter. In a flow mediated dilation test, the dilation degree of the vascular diameter in the brachial region is measured when an antebrachial region has been avascularized for a given period of time and then quickly released. The %FMD which represents the dilation degree is defined with the following formula:

$$\%\mathrm{FMD} = \frac{\mathrm{D_1 - D_0}}{\mathrm{D_0}} \times 100(\%) \qquad \dots (1)$$

where, Do signifies the largest vascular diameter at rest before the avascularization (which will be hereinafter called the "largest resting vascular diameter"), and $D_1$ signifies the largest vascular diameter after the release of the avascularization (which will be hereinafter called the "largest after-release vascular diameter").

The flow mediated dilation test (which will be hereinafter called a "%FMD measurement") is performed as follows. First, an ultrasonic probe is applied to the brachial region of a subject, and a tomographic image of a brachial artery at rest is portrayed by an ultrasonic scan to be saved in a video cassette recorder (VCR). Next, the antebrachial region of the subject is compressed with a cuff for avascularization for a given period of time, and after that, the tomographic image of the brachial artery after a quick release is portrayed to be also saved in the VCR. After a series of imagings is finished, the images at an appropriate point in time among the blood vessel images obtained in the resting state and after the release of the avascularization are selected, and the diameter of the blood vessel portrayed in these images is measured by an image analysis. With the largest resting vascular diameter Do and the largest after-release vascular diam-

eter $D_1$ measured by this operation, the %FMD is calculated based on the formula (1).
[Patent Document 1] Japanese Unexamined Patent Application Publication No. 2003-180690 ([0002])

DISCLOSURE OF THE INVENTION

PROBLEMS TO BE SOLVED BY THE INVENTION

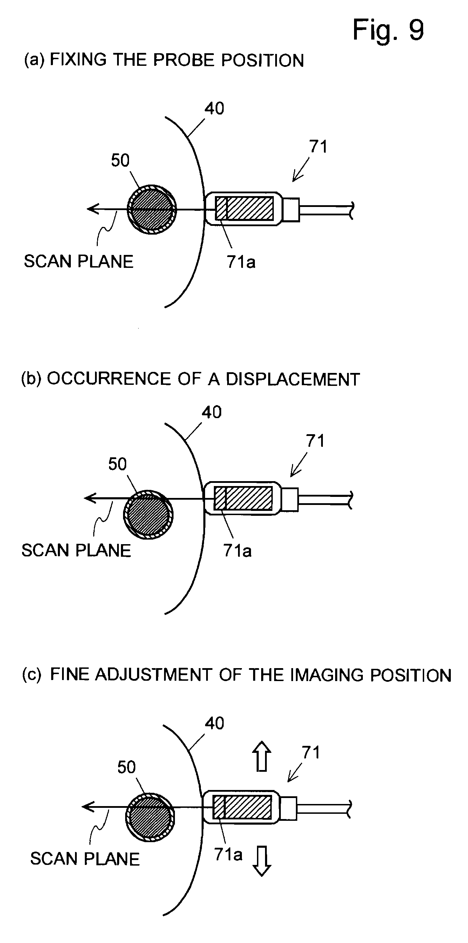

In performing a %FMD measurement as previously described, since the %FMD value is calculated based on the largest vascular diameters at rest and after the release of the avascularization, the probe needs to be positioned in advance so that the ultrasonic image of the section presenting the largest vascular diameter can be portrayed. Given this factor, before initiating the series of imagings as illustrated in Fig. 9(a) which presents a section orthogonal to the blood vessel's central axis, an operator applies a probe 71 to a predetermined position of the brachial region 40 of a subject. Then the operator locates the position and angle of the probe 71 with the largest vascular diameter, while looking at the ultrasonic image (which will be hereinafter called an "axial tomographic image"), which is portrayed by an ultrasonic scan, of the section parallel to the central axis of the blood vessel of the brachial artery 50. After that, with the probe 71 fixed at the located position, the vascular tomographic images at rest and after the release of the avascularization are taken.

However, when releasing the avascularization with the cuff, in some cases, the position between the probe 71 and the blood vessel 50 is displaced due to a rapid decompression (Fig. 9(b)). In such cases, the operator has to manually move the position and angle of the probe 71 to perform a fine adjustment (Fig. 9(c)), so that the largest vascular diameter is portrayed, which is cumbersome. In addition, such a fine adjustment is required to be promptly performed: if it takes time to perform a fine adjustment, an appropriate image cannot be portrayed while an image required for measuring the largest after-release vascular diameter should be saved, which might influence the analysis result.

Given this factor, the problems to be solved by the present invention is to provide an ultrasonograph capable of easily portraying a tomographic image presenting the largest vascular diameter and preferably usable for measuring the vascular diameter in a %FMD measurement or other measurements.

MEANS FOR SOLVING THE PROBLEMS

To solve the previously-described problem, the present invention provides an ultrasonograph including a vascular diameter measurement means for taking an ultrasonic tomography image of a blood vessel and then measuring a vascular diameter based on the image, including:

a) an ultrasonic probe, including an ultrasonic oscillator for performing an ultrasonic beam scan, the ultrasonic oscillator being capable of tilting or moving in parallel in a direction orthogonal to a scan direction of an ultrasonic beam;

b) a multi-section imaging means for making the ultrasonic oscillator tilt or move at predetermined intervals to perform an ultrasonic beam scan by the oscillator at each position in order to take an ultrasonic tomography image of the blood vessel at a plurality of sections;

c) an imaging position determiner for determining, based on the result of a measurement by the vascular diameter measurement means for each ultrasonic tomography image taken by the multi-section imaging means, a position where an image presenting an appropriate vascular diameter has been taken as a diagnostic imaging position; and

d) a largest vascular diameter acquisition means for making the ultrasonic oscillator tilt or move to the position determined by the imaging position determiner.

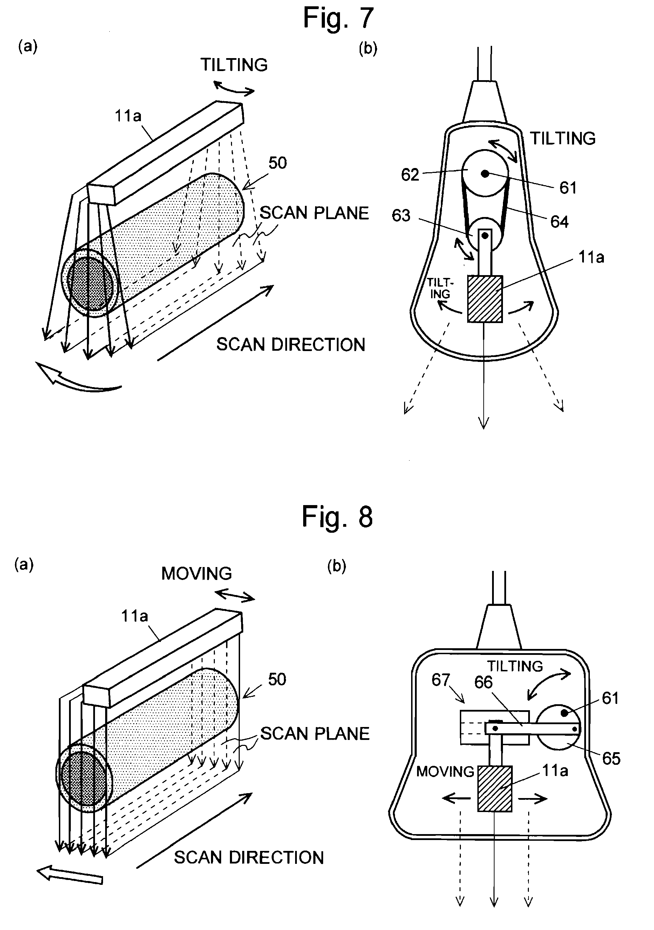

As the ultrasonic probe, any type of probe generally used as a probe for a four-dimensional imaging (or real-time three-dimensional imaging) or other type of probes can be used. Generally, such probes change the scan plane by tilting the oscillator 11a in the direction orthogonal to the scan direction of the ultrasonic beam as illustrated in Fig. 7(a): however, the ultrasonic probe according to the present invention may change the scan plane by moving the oscillator 11 a in the direction orthogonal to the scan direction of the ultrasonic beam as illustrated in Fig. 8(a).

EFFECTS OF THE INVENTION

With the ultrasonograph according to the present invention which has the aforementioned configuration, it is possible to automatically detect the appropriate imaging position and adjust the oscillator to the imaging position, which leads to a smooth measurement of the vascular diameter in a %FMD measurement and other measurements.

In the case where, as in a %FMD measurement, a blood vessel is imaged at the section in parallel with the axial direction and the vascular diameter is measured from the section image to be used in diagnosis, the ultrasonic probe is placed and used in such a manner that the axial direction of the blood vessel and the scan direction of the ultrasonic beam are substantially parallel. In this case, the ultrasonic oscillator is tilted or moved by the multi-section imaging means in the direction substantially orthogonal to the blood vessel's axial direction. In this instance, the imaging position determiner ascertains the position where the vascular diameter is largest as the diagnostic imaging position.

There are other kinds of measurements in which a blood vessel is imaged at a section orthogonal to the axial direction and the vascular diameter is measured from the tomographic image to be used in diagnosis. In such cases, the ultrasonic probe is placed in such a manner that the axial direction of the blood vessel and the scan direction of the ultrasonic beam are orthogonal. The ultrasonic oscillator is tilted by the multi-section imaging means in the direction substantially parallel to the blood vessel's axial direction. In this case, the imaging position determiner ascertains the position, as the diagnostic imaging position, where the vascular diameter is smallest in the vertical direction of the image (i.e. in the direction perpendicular to the skin).

The ultrasonograph according to the present invention may preferably have an avascularization means for compressing the body surface of a subject to avascularize for a given period of time a blood vessel to be diagnosed, then decompressing to release the avascularization, and simultaneously providing a decompression start signal. The multi-section imaging means may have the function of starting to image the plurality of sections in response to the decompression start signal. Therefore, the fine adjustment of the imaging position can be automatically started in response to the release of the avascularization, which further saves the operator's trouble in a %FMD measurement.

BRIEF DESCRIPTION OF THE DRAWINGS

Fig. 1 is a block diagram illustrating a schematic configuration of an ultrasonograph according to an embodiment of the present invention.

Fig. 2 is a sectional view illustrating the state where an ultrasonic probe according to the same embodiment is applied to the subject's brachial region, and illustrating a section parallel to the vascular axis of the brachial artery.

Fig. 3 is a sectional view illustrating the state where an ultrasonic probe according to the same embodiment is applied to the subject's brachial region, and illustrating a section orthogonal to the vascular axis of the brachial artery.

Fig. 4 is a flowchart illustrating the procedure of a %FMD measurement using the ultrasonograph according to the same embodiment.

Fig. 5 is a pattern diagram illustrating the state where a cuff and the ultrasonic probe are fixed to a subject.

Fig. 6 is a diagram illustrating an example of an axial tomographic image of a brachial artery portrayed on the monitor.

Fig. 7 is a pair of the diagrams explaining an ultrasonic probe having an oscillator tilting mechanism: (a) is a conceptual diagram illustrating a method for imaging a plurality of sections by the ultrasonic probe, and (b) is a sectional view illustrating a configuration example of the ultrasonic probe in a plane orthogonal to the ultrasonic beam's scan direction. Fig. 8 is a pair of the diagrams explaining an ultrasonic probe having an oscillator moving mechanism: (a) is a conceptual diagram illustrating a method for imaging a plurality of sections by the ultrasonic probe, and (b) is a sectional view illustrating a configuration example of the ultrasonic probe in a plane orthogonal to the ultrasonic beam's scan direction. Fig. 9 is a set of diagrams illustrating the positional relationship between a blood vessel and a probe in measuring a vascular diameter by a conventional ultrasonograph: (a) illustrates a state before an imaging is initiated, (b) illustrates a state in which a displacement has occurred, and (c) illustrates a fine adjustment process of an imaging position by an operator.

EXPLANATION OF NUMERALS

| 10 | Main Body Of An Ultrasonograph |
| 11 and 71 | Ultrasonic Probe |
| 11a and 71a | Ultrasonic Oscillator |
| 11b | Oscillator Tilting Mechanism |
| 12 | Transmission Controller |
| 13 | Tilting Controller |
| 14 | Ultrasonic Signal Processor |
| 15. | Display Processor |
| 16 | Monitor |
| 17 | Vascular Diameter Measuring Unit |
| 18 | Measurement Result Memory Unit |
| 19 | Image Storage Unit |
| 20 | Controller |
| 21 | Input Unit |
| 30 | Avascularization Unit |
| 31 | Cuff |
| 32 | Air Pipe |
| 33 | Press Pump |
| 34 | Avascularization Controller |
| 40 | Brachial Region |
| 50 | Brachial Artery |
| 51 | Intravascular Lumen |
| 52 | ...Vascular Wall |
| 61 | Motor's Rotational Axis |
| 62 and 63 | Pulley |
| 64 | Drive Belt |
| 65 | Eccentric Disk |
| 66 | Link |
| 67 | Slider |

BEST MODE FOR CARRYING OUT THE INVENTION

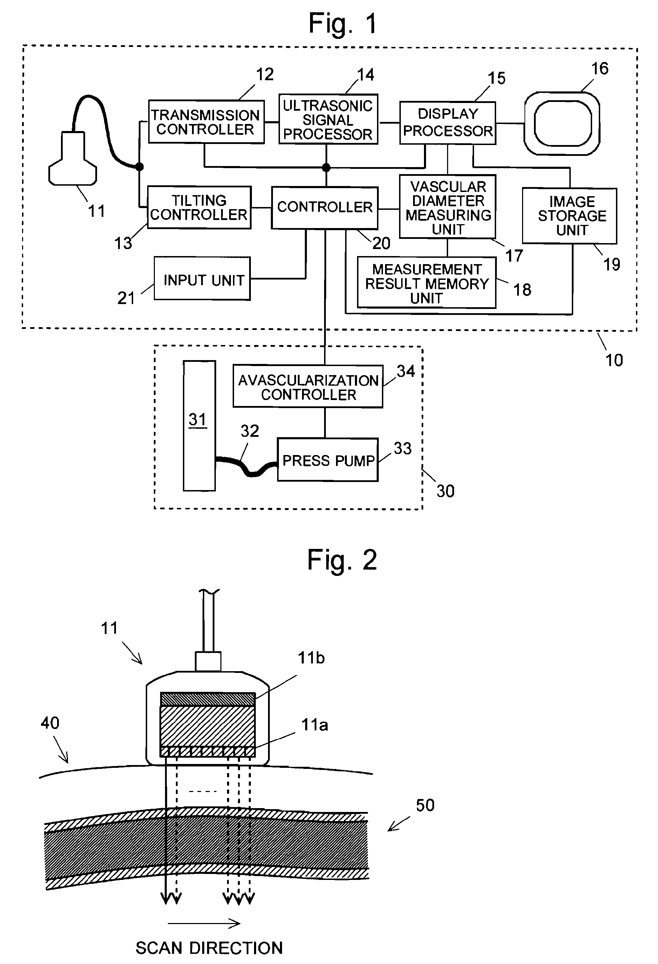

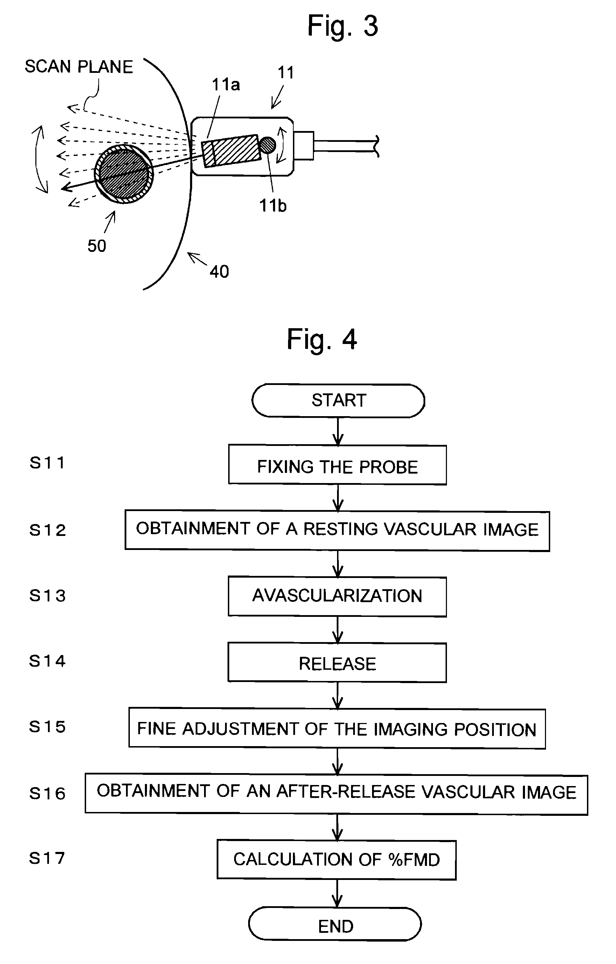

Hereinafter, the best mode for carrying out the present invention is described using an embodiment. Fig. 1 illustrates a schematic configuration of an ultrasonograph according to the present embodiment. Figs. 2 and 3 illustrate the state in which an ultrasonic probe according to the ultrasonograph of the present embodiment is applied to a subject's brachial region. Fig. 2 illustrates a section parallel to the brachial artery's axial direction, and Fig. 3 illustrates a section orthogonal to the brachial artery's axial direction.

An ultrasonic probe 11 transmits an ultrasonic wave to the inside of the body of a subject and simultaneously receives the ultrasonic wave reflected inside the subject's body to convert it into an electrical signal. The ultrasonic probe 11 according to the present embodiment incorporates an oscillator array 11a and an oscillator tilting mechanism 11b. The oscillator array 11a is composed of a number of ultrasonic oscillators which are one-dimensionally arranged and performs an ultrasonic beam scan by a linear scan method, and the oscillator tilting mechanism 11b tilts the oscillator array 11a around a virtual rotational axis which is parallel to the oscillators' array direction. That is, the oscillator tilting mechanism 11b tilts the oscillator array in the direction orthogonal to the ultrasonic beam's scan direction. As illustrated in Fig. 7(b) for example, the oscillator tilting mechanism 11b may transmit the tilting force of the motor (not shown) to the oscillator array 11a with two pulleys 62 and 63 respectively assembled at the rotational axis 61 and the oscillator array 11a, with a drive belt 64 hung between the two pulleys 62 and 63, and with other elements.

A transmission controller 12 controls the ultrasonic beam scan by the oscillator array 11a, and a tilting controller 13 controls the tilting of the oscillator 11a by the oscillator tilting mechanism 11b.

The electrical signal of the reflected ultrasonic wave provided by the ultrasonic probe 11 is fed to an ultrasonic signal processor 14. The ultrasonic signal processor 14 converts this electrical signal into image data, and further performs a data processing to the image data appropriate for displaying the image, such as a phased summation, gain adjustment or logarithmic compression. After the processing, the data is delivered from the ultrasonic signal processor 14 to a display processor 15. In the display processor 15, an electrical signal to be displayed in a monitor 16 is created from the image data, and the electrical signal is displayed on the monitor 16. The operations described so far are repeated at predetermined intervals, and the ultrasonic images are displayed on the monitor 16 as a moving image. On the other hand, the moving image may be saved in an image storage unit 19 composed of a VCR for a given period of time in response to the operator's instruction.

A vascular diameter measuring unit 17 performs the detection of a vascular wall and the measurement of the distance between the vascular walls by using a predetermined image analysis algorithm to the blood vessel's tomographic image stored in the memory of the display processor 15 in order to derive the vascular diameter. A measurement result memory unit 18 stores the measurement result by the vascular diameter measuring unit 17.

Each of the aforementioned units is controlled by a controller 20 which is composed of a central processing unit (CPU) or the like. To the controller 20, an input unit 21 is connected which is composed of a pointing device such as a mouse or trackball, keyboard, or other units for allowing the user to enter the setting, instruction, etc.

In addition, the controller 20 is connected to an avascularization unit 30 for avascularizing the subject's brachial artery. The avascularization unit 30 has a cuff 31 which is wrapped around the subject's antebrachial region to be used, an air pipe 32 and a press pump 33 for pumping air into the cuff 31, and an avascularization controller 34 for controlling these units. The press pump 33 has an electromagnetic valve for discharging the air. When an avascularization is released, the electromagnetic valve is opened, and simultaneously a decompression start signal for indicating the initiation of the decompression of the cuff 31 is provided to the controller 20 in the main body 10 of the ultrasonograph. Additionally, an instruction from the operator can be given to the avascularization controller 34 through the input unit 21 provided in the main body 10 of the ultrasonograph.

Next, a procedure of a %FMD measurement using the ultrasonograph according to the present embodiment will be described by using the flowchart of Fig. 4.

(1) Fixing the Probe (Step S 11)

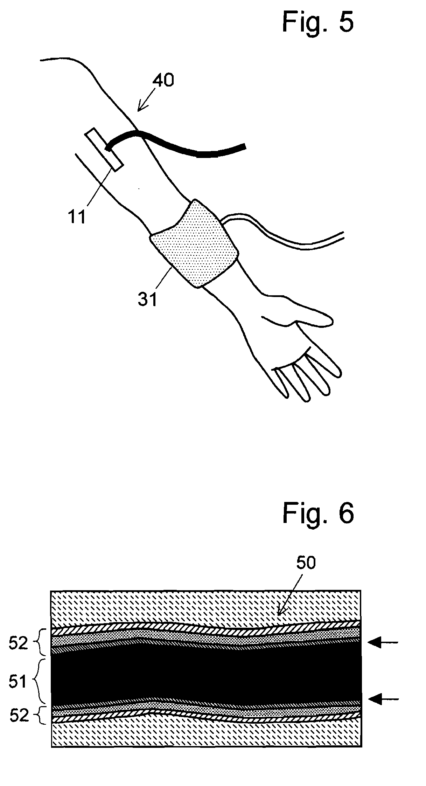

First, the cuff 31 is wrapped around the antebrachial region of the subject and the subject is kept at rest for fifteen minutes. Then, the probe 11 is applied to a predetermined position of the brachial region 40 (Fig. 5). At this point in time, the probe 11 is set in such a manner that the array direction of the oscillator array 11a (i.e. the scan direction of the ultrasonic beam) and the axial direction of the arm are parallel. Subsequently, the ultrasonic beam scan by the oscillator array 11a is performed and an axial tomographic image of the brachial artery 50 (Fig. 6) is portrayed on the monitor 16. The operator searches for the position where the diameter of the portrayed blood vessel 50 is largest by manually moving the position and angle of the probe 11 while looking at the image on the monitor 16, and fixes the probe 11 with a fixture (not shown) so that the probe will not move from the position.

(2) Obtainment of a Resting Vascular Image (Step S12)

During the aforementioned state, ultrasonic images are taken, and the operator performs a predetermined operation through the input unit 21 to save the images in the image storage unit 19 for a given period of time. The image taken at this point in time will be hereinafter called a "resting vascular image."

(3) Avascularization (Step S13) and Release (Step S14)

After the obtainment of the resting vascular image is completed, the avascularization of the brachial artery by the cuff 31 is initiated. In the avascularization unit 30, the avascularization controller 34 controls the press pump 33 to pump air into the cuff 31 which is wrapped around the subject's antebrachial region and halts the air supply when the pressure reaches 250mmHg. Five minutes later, the avascularization controller 34 opens the electromagnetic valve provided in the press pump 33 in order to discharge the air to quickly reduce the pressure. Upon opening the electromagnetic valve, the avascularization controller 34 also transmits a signal (i.e. decompression start signal) for indicating the initiation of the decompression to the controller 20 in the main body 10 of the ultrasonograph.

(4) Fine Adjustment of the Imaging Position (Step S15)

Subsequently, the fine adjustment process of the imaging position, which is a feature of the present invention, will be described. When the decompression start signal is provided to the controller 20, the controller 20 directs the tilting controller 13 and the transmission controller 12 to perform a fine adjustment of the imaging position. As illustrated in Fig. 3, the tilting controller 13 controls the oscillator tilting mechanism 11b to tilt the oscillator array 11a at given angular intervals, and the transmission controller 12 controls the oscillator array 11a to perform an ultrasonic scan by a linear scan method at each tilting angle. Consequently, axial tomographic images of the brachial artery 50, whose sectional positions are slightly different, are sequentially taken and portrayed on the monitor 16.

In the vascular diameter measuring unit 17, using the image data (or brightness value of each picture element) for an image display stored in the memory of the display processor 15, the boundary positions (which are indicated by the arrows in Fig. 6) between the intravascular lumen 51 and two vascular walls (i.e. the closer wall and the far wall from the skin) are detected based on the brightness distribution. The distance between the two boundary positions is measured as the vascular diameter. The measurement value obtained for each image is associated with the angular position of the oscillator array 11 a when the image is taken, and then is stored in the measurement result memory unit 18. For such a detection of the vascular walls and measurement of the vascular diameter, a conventionally-known algorithm can be used for example.

When taking a predetermined number of images and measuring the vascular diameter are completed, the value of the vascular diameter measured for each image is read out from the measurement result memory unit 18, and the measurement values are compared. Accordingly, the image presenting the largest vascular diameter is specified and the angular position of the oscillator array 11 a when the image was taken is determined as the imaging position capable of capturing the largest vascular diameter. According to this, the oscillator array 11a is tilted to the imaging position by the control of the tilting controller 13 and subsequent imagings are performed at the imaging position.

### (5) Obtainment of an After-Release Vascular Image (Step S16)

When the aforementioned imaging position's fine adjustment is completed, the operator orders the images to be saved through the input unit 21, so that the tomographic image of the brachial artery 50 portrayed by the probe 11 is stored in the image storage unit 19 after the release of the avascularization and before a predetermined period of time elapses to complete the imaging. The image taken at this point in time will be hereinafter called an "after-release vascular image."

### (6) Calculation of the %FMD (Step S17)

After a series of imaging processes as previous described is completed, the operator operates the input unit 21 to play the resting vascular images stored in the image storage unit 19. When the operator additionally performs a predetermined operation to freeze the image at an appropriate point in time and instructs the measurement of the vascular diameter, the vascular diameter measuring unit 17 measures the diameter of the blood vessel 50 portrayed in the image based on the image's brightness distribution in the same manner as in Step S 15, and stores the obtained value as the largest resting vascular diameter Do in the measurement result memory unit 18. Such a freeze of the image and measurement of the vascular diameter may be repeated multiple times and the obtained values may be averaged to obtain the result with higher accuracy. Furthermore, in the same manner, the largest after-release vascular diameter $D_1$ is measured from the after-release vascular image stored in the image storage unit 19. From these values of Do and $D_1$, the %FMD is calculated using the aforementioned formula (1) and displayed on the monitor 16.

As just described, with the ultrasonograph according to the present embodiment, the oscillator array 11a is tilted by the ultrasonic probe 11 having the oscillator tilting mechanism 11b, and simultaneously the angular position in which the largest vascular diameter can be portrayed is located based on the measurement result by the vascular diameter measuring unit 17, which achieves an automatic fine adjustment of the imaging position. Accordingly, the operator is no longer required, as before, to manually fine adjust the position and angle of the probe 11, and the image appropriate for the measurement of the largest after-release vascular diameter can be assuredly portrayed and stored. Since such a fine adjustment of the imaging position is automatically initiated in response to the decompression start signal transmitted from the avascularization unit 30, the operator's trouble for a %FMD measurement can be further saved, which leads to a smooth measurement.

The best mode for carrying out the present invention has been described so far using an embodiment. However, it should be noted that the present invention is not limited to the previously described embodiment, and a variety of modifications are allowed within the spirit of the present invention. For example, the imaging position's fine adjustment by the ultrasonograph according to the present invention can be used for correcting a displacement between the probe and a blood vessel in releasing the avascularization as in the aforementioned embodiment, and additionally it can also be used for the position adjustment in fixing the ultrasonic probe to the brachial region before starting a series of measurements. In such a case, when the probe is fixed to the brachial region with a fixture as previously described, only a rough position adjustment is performed. After that, a fine adjustment is performed by using the multi-section imaging means, imaging position determiner, and largest vascular diameter acquisition means according to the present invention. In this case, a fine adjustment of the imaging position is automatically started in response to a decompression start signal from the avascularization unit as in the aforementioned embodiment, and additionally, the imaging position search may be started in response to the operator's instruction.

The ultrasonic probe of the ultrasonograph according to the present invention may have an oscillator moving mechanism, in place of the oscillator tilting mechanism as in the aforementioned embodiment, for moving the oscillator in the direction orthogonal to the ultrasonic beam's scan direction at predetermined intervals. For example, the oscillator moving mechanism may be configured as shown in Fig. 8(b), in which the tilting of a motor (not shown) is converted into the moving motion of the oscillator 11 a with an eccentric disk 65, which is attached to the motor's rotational axis 61, a link 66 and a slider 67.

### Claims

1. An ultrasonograph including a vascular diameter measurement means for taking an ultrasonic tomography image of a blood vessel and then measuring a vascular diameter based on the image, comprising:

   a) an ultrasonic probe, including an ultrasonic oscillator for performing an ultrasonic beam scan, the ultrasonic oscillator being capable of tilting or moving in parallel in a direction orthogonal to a scan direction of an ultrasonic beam;
   b) a multi-section imaging means for making the ultrasonic oscillator tilt or move at predetermined intervals to perform an ultrasonic beam scan by the oscillator at each position in order to take an ultrasonic tomography image of the blood vessel at a plurality of sections;

c) an imaging position determiner for determining, based on a result of a measurement by the vascular diameter measurement means for each ultrasonic tomography image taken by the multi-section imaging means, a position where an image presenting an appropriate vascular diameter has been taken as a diagnostic imaging position; and

d) a largest vascular diameter acquisition means for making the ultrasonic oscillator tilt or move to the position determined by the imaging position determiner.

2. The ultrasonograph according to claim 1, wherein:

the ultrasonic probe is placed in such a manner that an axial direction of a blood vessel to be diagnosed and a scan direction of the ultrasonic beam by the ultrasonic oscillator are substantially in parallel; and

the imaging position determiner determines a position where an image presenting a largest vascular diameter has been taken as the diagnostic imaging position.

3. The ultrasonograph according to either claim 1 or 2, further comprising

e) an avascularization means for compressing a body surface of a subject to avascularize a blood vessel to be diagnosed, decompressing after a predetermined period of time to release the avascularization, and simultaneously providing a decompression start signal, wherein the multi-section imaging means starts imaging the plurality of sections in response to the decompression start signal.

## Fig. 1

## Fig. 2

SCAN DIRECTION

# Fig. 3

# Fig. 4

```
                    ┌──────────────┐
                    │    START     │
                    └──────┬───────┘
                           ↓
S11         ┌──────────────────────────────┐
            │      FIXING THE PROBE         │
            └──────────────┬───────────────┘
                           ↓
S12   ┌────────────────────────────────────────────┐
      │  OBTAINMENT OF A RESTING VASCULAR IMAGE      │
      └──────────────────────┬─────────────────────┘
                             ↓
S13         ┌──────────────────────────────┐
            │       AVASCULARIZATION        │
            └──────────────┬───────────────┘
                           ↓
S14         ┌──────────────────────────────┐
            │           RELEASE             │
            └──────────────┬───────────────┘
                           ↓
S15   ┌────────────────────────────────────────────┐
      │  FINE ADJUSTMENT OF THE IMAGING POSITION     │
      └──────────────────────┬─────────────────────┘
                             ↓
S16 ┌──────────────────────────────────────────────────┐
    │ OBTAINMENT OF AN AFTER-RELEASE VASCULAR IMAGE      │
    └────────────────────────┬─────────────────────────┘
                             ↓
S17         ┌──────────────────────────────┐
            │      CALCULATION OF %FMD       │
            └──────────────┬───────────────┘
                           ↓
                    ┌──────────────┐
                    │     END      │
                    └──────────────┘
```

Fig. 5

Fig. 6

# Fig. 7

(a)

TILTING

11a

50

SCAN PLANE

SCAN DIRECTION

(b)

TILTING

62

61

63

64

11a

TILT-
ING

# Fig. 8

(a)

MOVING

11a

50

SCAN PLANE

SCAN DIRECTION

(b)

TILTING

67    66

61

65

MOVING    11a

# Fig. 9

(a) FIXING THE PROBE POSITION

40

50

71

SCAN PLANE

71a

(b) OCCURRENCE OF A DISPLACEMENT

40

50

71

SCAN PLANE

71a

(c) FINE ADJUSTMENT OF THE IMAGING POSITION

40

50

71

SCAN PLANE

71a

## INTERNATIONAL SEARCH REPORT

| International application No. |
| --- |
| PCT/JP2006/311776 |

**A. CLASSIFICATION OF SUBJECT MATTER**
*A61B8/08*(2006.01)i

According to International Patent Classification (IPC) or to both national classification and IPC

**B. FIELDS SEARCHED**

Minimum documentation searched (classification system followed by classification symbols)
A61B8/08

Documentation searched other than minimum documentation to the extent that such documents are included in the fields searched
| | | | |
| --- | --- | --- | --- |
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| A | JP 2006-81640 A (GE Medical Systems Global Technology Co. LLC.), 30 March, 2006 (30.03.06), Particularly, Par. Nos. [0050], [0051] (Family: none) | 1-3 |
| A | JP 2001-299752 A (Aloka Co., Ltd.), 30 October, 2001 (30.10.01), Particularly, Figs. 2, 3 & EP 1123687 A2 & US 6673020 B2 | 1-3 |
| A | JP 2006-6686 A (Aloka Co., Ltd.), 12 January, 2006 (12.01.06), Full text; all drawings (Family: none) | 1-3 |

☐ Further documents are listed in the continuation of Box C. ☐ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| 03 October, 2006 (03.10.06) | 10 October, 2006 (10.10.06) |

| Name and mailing address of the ISA/ | Authorized officer |
| --- | --- |
| Japanese Patent Office | |
| Facsimile No. | Telephone No. |

Form PCT/ISA/210 (second sheet) (April 2005)

**REFERENCES CITED IN THE DESCRIPTION**

**Patent documents cited in the description**

- JP 2003180690 A